(19) Europäisches Patentamt
European Patent Office
Office européen des brevets

(11) Veröffentlichungsnummer: **0 325 892 B1**

(12) **EUROPÄISCHE PATENTSCHRIFT**

(45) Veröffentlichungstag der Patentschrift: **03.02.93**

(51) Int. Cl.[5]: **C07C 209/36**, C07C 211/52

(21) Anmeldenummer: **88810892.5**

(22) Anmeldetag: **22.12.88**

# (54) Verfahren zur Herstellung von halogenierten aromatischen primären Aminen.

(30) Priorität: **31.12.87 CH 5126/87**

(43) Veröffentlichungstag der Anmeldung:
**02.08.89 Patentblatt 89/31**

(45) Bekanntmachung des Hinweises auf die Patenterteilung:
**03.02.93 Patentblatt 93/05**

(84) Benannte Vertragsstaaten:
**CH DE FR GB IT LI NL**

(56) Entgegenhaltungen:
**DE-A- 2 441 650**

**CHEMICAL ABSTRACTS, Band 97, 30. August 1982, Seite 604, Zusammenfassung Nr. 72059b, Columbus, Ohio, US; & CS-A-196 476**

**HELVETICA CHIMICA ACTA, Band 42, 16. März 1959, Seiten 407-416; G. TRÜMPLER et al.: "Katalytische und elektrolytische Reduktion von Cyanamid an feinverteiltem Nickel"**

(73) Patentinhaber: **CIBA-GEIGY AG**
**Klybeckstrasse 141**
**CH-4002 Basel(CH)**

(72) Erfinder: **Baumeister, Peter**
**St.Annaweg 24**
**CH-4113 Flüh(CH)**
Erfinder: **Scherrer, Wilfried**
**Feldstrasse 10**
**CH-4416 Bubendorf(CH)**

**EP 0 325 892 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von halogenierten aromatischen primären Aminen durch katalytische Hydrierung von halogenierten aromatischen Nitroverbindungen in Gegenwart von Raney-Nickel und einem Formamidinsalz als Dehalogenierungsinhibitor.

Es ist bekannt, dass man durch katalytische Hydrierung von halogenierten, aromatischen Nitroverbindungen halogenierte aromatische primäre Amine erhält. Die Hydrierung führt auch zu einer Dehalogenierung und damit zu schwer trennbaren Gemischen halogenfreier und halogenierter aromatischer primärer Amine.

In der DE-AS 2 441 650 wird ein Hydrierungsverfahren in Gegenwart von Raney-Nickel beschrieben, bei dem Dicyandiamid, Cyanamid oder Calciumcyanamid als Dehalogenierungsinhibitor verwendet wird. Es wird auch offenbart, dass das Dicyandiamid teilweise hydriert wird. Da das Reaktionsgemisch basisch ist, kann aber kein Formamidinsalz als stabiles Zwischenprodukt gebildet werden. G. Trümpler et al. beschreiben nämlich bereits in Helv. Chim. Acta, 39, S. 407-416 (1959), dass bei der katalytischen Hydrierung von Cyanamid mit Raney-Nickel nur unter schwach sauren Bedingungen (pH ~ 6) Formamidinsalze als primäre Reaktionsprodukte gebildet werden.

Es wurde nun gefunden, dass Formamidinsalze die Dehalogenierung bei der Hydrierung von halogenierten aromatischen Nitroverbindungen auch bei erhöhten Temperaturen wirksam unterdrücken. Die gewünschten halogenierten aromatischen Amine werden im Vergleich ohne Zusatz in höheren, unter günstigen Bedingungen in praktisch quantitativen Ausbeuten erhalten, wobei der Gehalt an Nebenprodukten bei optimalen Bedingungen wesentlich unter 1 Gew.-% betragen kann. Die Katalysatoraktivität wird nicht beeinträchtigt, so dass das Raney-Nickel wiederholt eingesetzt werden kann. Die Hydriergeschwindigkeit wird nicht nennenswert beeinflusst. Das Verfahren ist zur Durchführung im technischen Massstab geeignet.

Gegenstand der Erfindung ist ein Verfahren zur Herstellung von halogenierten aromatischen primären Aminen durch katalytische Hydrierung von halogenierten aromatischen Nitroverbindungen in Gegenwart von Raney-Nickel bei einem Druck von 0,1 bis 100 bar und bei einer Temperatur von 30 bis 150 °C in einem inerten Lösungsmittel und in Gegenwart eines Inhibitors gegen eine Dehalogenierung, das dadurch gekennzeichnet ist, dass der Inhibitor ein Formamidinsalz ist.

Als Nitroverbindungen kommen z.B. solche der allgemeinen Formel

$$(Y)_x\text{—}A\text{—}(NO_2)_y$$

in Frage, worin x und y unabhängig voneinander eine Zahl von 1 bis 6, bevorzugt 1 bis 3 und besonders 1 oder 2 bedeuten, Y für Halogen, besonders für F, Cl und Br, steht, und A einen aromatischen Rest mit 6-18 C-Atomen darstellt, der noch weitere Substituenten enthalten kann. Y bedeutet insbesondere Cl oder Br.

Der aromatische Rest enthält bevorzugt 6 bis 14, besonders 6-10 C-Atome und stellt insbesondere einen aromatischen Kohlenwasserstoffrest dar. Beispiele für aromatische Kohlenwasserstoffe, von denen sich der Rest ableiten kann, sind zum Beispiel: Benzol, Naphthalin, Phenanthren, Biphenyl, Indan, durch hydrierstabile Brückengruppen $Y^1$ verknüpftes Benzol, wie z.B.

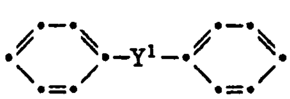 ,

worin $Y^1$ $C_1$-$C_4$-Alkylen, $C_1$-$C_6$-Alkyliden, -O-, -S-, $-SO_2-$ oder -CO-sein kann.

Geeignete weitere Substituenten für den Rest A sind z.B. $C_1$-$C_4$-Alkyl, -Halogenalkyl, -Alkoxy, -Halogenalkoxy, oder -Alkylthio, $C_5$-oder $C_6$-Cycloalkyl, Cyano, Hydroxyl, $C_1$-$C_8$-Acyl oder -Acyloxy, -COOH, $-SO_3H$, -COOM und $-SO_3M$, worin M für ein Alkalimetallkation, z.B. $Na^{\oplus}$, oder Ammonium steht, $-SO_3R^3$ oder $-CO_2R^3$, worin $R^3$ $C_1$-$C_6$-Alkyl, Phenyl oder Benzyl ist, $-NH_2$, $C_1$-$C_8$-Alkyl- oder -Dialkylamino, $C_1$-$C_8$-Acylamino oder $C_1$-$C_8$-Aminocarbonyl. Einige Beispiele sind Methyl, Ethyl, n-oder iso-Propyl, n-, i- oder t-Butyl, Methyloxy, Ethyloxy, Chlorethyloxy, Methylthio, Chlormethyl, Fluormethyl, Trifluormethyl, Trichlormethyl, Cyclopentyl, Cyclohexyl, Acetyl, Propionyl, Acetyloxy, Methoxysulfonyl, Methoxy- oder Ethoxycarbonyl, Methylamino, Dimethylamino, Acetylamino, Aminocarbonyl und Dimethylaminocarbonyl.

Beispiele für Nitroverbindungen sind o-, m- und p-Chlor- oder Bromnitrobenzol, 2,3-, 3,4-, 2,4-, 2,6-, 3,5-

und 2,5-Dichlor- oder Dibromnitrobenzol, 2,3,4- und 2,3,5-Trichlornitrobenzol, 2-Chlor-3-Bromnitrobenzol, 1-Chlor- oder 1-Brom-2,4-dinitrobenzol, 3,4-Dichlor-1,6-dinitrobenzol, 2,4-Dichlor-1,6-dinitrobenzol, 1-Chlor-2,4,6-trinitrobenzol, 4- oder 6-Chlor-1-methyl-2-nitrobenzol, 2-oder 6-Brom-1-methyl-4-nitrobenzol, 4-oder 6-Chlor-1-ethyl-2-nitrobenzol, 2- oder 3-Brom-1-ethyl-4-nitrobenzol, 4- oder 6-Chlor-1-methoxy-4-nitrobenzol, 4-oder 6-Chlor-2-nitrophenol, 1-Chlor- oder 1-Brom-2-nitronaphthalin, 1-Chlor- oder 1-Brom-2,7-dinitronaphthalin, 2-Chlor-4-nitrodiphenyl, 2,2′-Dichlor-4,4′-dinitro-diphenyl, 2-Brom-4,4′-dinitrobenzophenon, Bis(3,3′-dichlor-4,4′-dinitrophenyl)methan und 3,3′-Dichlor-4,4′-dinitrodiphenylether.

Das Formamidinsalz wird vorteilhaft in einer Menge von 0,1 bis 30 Mol-%, bevorzugt 0,1 bis 20 Mol-% und besonders 0,5 bis 15 Mol-% verwendet, bezogen auf die Nitroverbindung.

Das Formamidinkation kann mit einem, zwei oder drei Kohlenwasserstoffresten substituiert sein, die vorzugsweise 1 bis 18, besonders 1-12, und insbesondere 1 bis 6 C-Atome enthalten. Das Anion des Formamidinsalzes kann sich z.B. von aliphatischen oder aromatischen Carbonsäuren, bevorzugt Mono- oder Dicarbonsäuren ableiten, die vorzugsweise 1 bis 18, besonders 1-8, und insbesondere 1 bis 4 C-Atome enthalten.

Eine bevorzugte Ausführungsform ist jene worin das Formamidinsalz der Formel I

$$[R^3N{=}\overset{H}{C}{-}\overset{H}{N}R^1R^2]^{\oplus}_{n}X^{n\ominus} \qquad (I)$$

entspricht, worin $R^1$, $R^2$ und $R^3$ unabhängig voneinander für H stehen, oder lineares oder verzweigtes $C_1$-$C_{12}$-Alkyl, $C_5$- oder $C_6$-Cycloalkyl, $C_6$-$C_{18}$-Alkylcycloalkyl, $C_6$-$C_{10}$-Cycloalkylalkyl, $C_7$-$C_{18}$-Alkylcycloalkylalkyl, wobei das Cycloalkyl 5 oder 6 Ring-C-Atome enthält, $C_6$-$C_{10}$-Aryl, $C_7$-$C_{18}$-Alkaryl, $C_7$-$C_{12}$-Aralkyl oder $C_7$-$C_{18}$-Alkaralkyl oder $R^1$ und $R^2$ zusammen Tetra- oder Pentamethylen oder 3-Oxa-1,5-Pentylen darstellen, X das Anion einer Säure, besonders einer $C_1$-$C_{18}$-Mono- oder -Dicarbonsäure bedeutet, und n 1 oder 2 ist.

$R^1$, $R^2$ und $R^3$ enthalten als Alkyl vorzugsweise 1 bis 6 und besonders 1 bis 4 C-Atome. Beispiele sind Methyl, Ethyl und die Isomeren von Propyl, Butyl, Pentyl, Hexyl, Heptyl, Octyl, Decyl und Dodecyl. $R^1$, $R^2$ und $R^3$ sind als Cycloalkyl z.B. Cyclopentyl und Cyclohexyl. Als Alkylcycloalkyl enthalten $R^1$, $R^2$ und $R^3$ bevorzugt 6 bis 12, besonders 6 bis 10 C-Atome. Beispiele sind Methylcyclopentyl, Methylcyclohexyl, Ethylcyclohexyl und n-Propylcyclohexyl. $R^1$, $R^2$ und $R^3$ sind als Cycloalkylalkyl besonders Cyclopentylmethyl oder Cyclohexylmethyl. Als Alkylcycloalkylalkyl enthalten $R^1$, $R^2$ und $R^3$ bevorzugt 7 bis 12 C-Atome und sind insbesondere $C_1$-$C_4$-Alkylcyclopentylmethyl oder -cyclohexylmethyl. $R^1$, $R^2$ und $R^3$ in der Bedeutung von Aryl kann z.B. Naphthyl und besonders Phenyl sein. In den Aryl enthaltenden Resten für $R^1$, $R^2$ und $R^3$ ist das Aryl besonders Phenyl. $R^1$, $R^2$ und $R^3$ in der Bedeutung von Alkaryl enthalten bevorzugt 7 bis 12 C-Atome und stellen besonders $C_1$-$C_4$-Alkylphenyl dar. $R^1$, $R^2$ und $R^3$ als Aralkyl sind besonders Benzyl und Phenylethyl. $R^1$, $R^2$ und $R^3$ als Alkaralkyl enthalten bevorzugt 8 bis 14 C-Atome und stellen besonders $C_1$-$C_4$-Alkylbenzyl dar.

Bei $R^1$, $R^2$ und $R^3$ handelt es sich bevorzugt um H oder aliphatische beziehungsweise cycloaliphatische Reste.

In einer bevorzugten Ausführungsform stellen $R^1$, $R^2$ und $R^3$ unabhängig voneinander H oder $C_1$-$C_6$-Alkyl, besonders H, Methyl oder Ethyl und insbesondere jeweils H dar.

In Formel I steht n vorzugsweise für 1.

X als Anion einer Mono- oder Dicarbonsäure enthält bevorzugt 1-18, besonders 1-12 und insbesondere 1 bis 8 C-Atome. Es kann sich um aliphatische oder aromatische Carbonsäuren handeln, die z.B. den Formeln

$R^4COOH$ oder $R^5(COOH)_2$

entsprechen können, worin $R^4$ lineares oder verzweigtes Alkyl mit besonders 1 bis 6 C-Atomen, Cyclohexyl, Phenyl oder Benzyl ist, und $R^5$ eine direkte Bindung, lineares oder verzweigtes $C_2$-$C_6$-Alkylen, Cyclohexylen oder Phenylen darstellt.

Insbesondere stellt X in Formel I das Anion einer aliphatischen Mono-oder Dicarbonsäure mit bevorzugt 1 bis 4 C-Atomen dar. Besonders steht X für $\frac{2\ominus}{2}$ die Anionen $(COO)_2^{2\ominus}$, $HCOO^{\ominus}$, $CH_3COO^{\ominus}$, $CH_3CH_2COO^{\ominus}$, $CH_3CH_2CH_2COO^{\ominus}$ oder $CH_2(COO)_2^{2\ominus}$, und ganz besonders für $CH_3COO^{\ominus}$.

Insbesondere handelt es sich bei dem Formamidinsalz um Formamidinacetat.

Das Formamidinsalz kann als solches dem Reaktionsgemisch beigegeben werden, oder nach einer bekannten Methode in situ vor der Hydrierung erzeugt werden, z.B. indem man ein gegebenenfalls substituiertes Cyanamid in Gegenwart von Raney-Nickel und einer Säure hydriert.

Die in situ Bildung des gegebenenfalls substituierten Formamidinsalzes erfolgt so schnell, dass man diese Reaktion in Gegenwart einer halogenierten aromatischen Nitroverbindung durchführen kann. Es kann hierbei ein Teil der Nitroverbindung zugegeben und der Rest nachdosiert werden, oder es kann die gesamte Menge Nitroverbindung mitvorgelegt werden.

Die Menge an Raney-Nickel beträgt bevorzugt 0,5 bis 20 Gew.-%, besonders 1 bis 10 Gew.-%, bezogen auf die Nitroverbindung.

Die Hydrierung wird in Substanz oder in einem inerten Lösungsmittel durchgeführt. Geeignete Lösungsmittel sind z.B. Alkanole (Methanol, Ethanol, Propanol, Butanol, Methoxy- oder Ethoxyethanol), Ester (Dibutylether, t-Butylmethylether, Tetrahydrofuran, Dioxan, Ethylenglykoldimethylether, Diethylenglykoldimethylether), Amide und Lactame (Dimethylformamid, Dimethylacetamid, N-Methylpyrrolidon), Ester und Lactone (Essigsäureethylester, $\gamma$-Butyrolacton), Kohlenwasserstoffe (Pentan, Hexan, Methylcyclohexan, Benzol, Toluol, Xylol, Chlorbenzol), Wasser. Bei der Hydrierung in Substanz ist das bei der Hydrierung entstehende halogenierte aromatische Amin das Lösungsmittel.

In einer bevorzugten Ausführungsform wird als Lösungsmittel ein $C_1$-$C_4$-Alkanol alleine oder im Gemisch mit Wasser verwendet. Insbesondere wird Methanol verwendet.

Die Reaktionstemperatur beträgt vorteilhaft 50 bis 120 °C. Der Druck beträgt vorzugsweise 1 bis 30 bar. Die Reaktionszeit richtet sich im wesentlichen nach den Reaktionsbedingungen und beträgt im allgemeinen weniger als zwei Stunden.

Das erfindungsgemässe Verfahren kann so durchgeführt werden, dass man in einem Autoklaven die Nitroverbindung, das Raney-Nickel, das Lösungsmittel und das Formamidinsalz einträgt und die Luft zuerst durch Stickstoff und diesen dann durch Wasserstoff verdrängt. Dann wird der Autoklav verschlossen, Wasserstoff bis zum gewünschten Druck aufgepresst und auf die Reaktionstemperatur erhitzt. Nach Beendigung der Reaktion wird das Reaktionsgemisch vom Katalysator abgetrennt. Danach kann man das Reaktionsprodukt vom Reaktionswasser und Lösungsmittel abtrennen und durch Destillation oder Umkristallisation weiter reinigen. Die primären aromatischen Amine sind bekannterweise Zwischenprodukte zur Herstellung von Farbstoffen.

Die nachfolgenden Beispiele erläutern die Erfindung näher.

Beispiel 1:

40,8 g 1-Chlor-2,4-dinitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 60 °C wird hydriert. Die Hydrierzeit beträgt $1\frac{1}{4}$ Stunden. Man erhält in quantitativer Ausbeute 1-Chlor-2,4-Diaminobenzol mit einer Reinheit von 99 % (flüssigchromatographische Analyse, <Nachweis als 1-Chlor-2,4-diacetamidobenzol>.

Beispiel 2:

39,8 g 1-Chlor-2-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 90 °C wird hydriert. Die Hydrierzeit beträgt $1\frac{1}{2}$ Stunden. Man erhält in quantitativer Ausbeute 1-Chlor-2-aminobenzol mit einer Reinheit von 99,4 % (gaschromatographische Analyse).

Beispiel 3:

39,8 g 1-Chlor-4-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 12 bar und einer Temperatur von 80 °C wird hydriert. Die Hydrierzeit beträgt $1\frac{1}{2}$ Stunden. Man erhält in quantitativer Ausbeute 1-Chlor-4aminobenzol mit einer Reinheit von 99,7 % (gaschromatographische Analyse).

Beispiel 4:

39,8 g 1-Chlor-3-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft durch Stickstoff und dann durch Wasserstoff verdrängt. Bei einem Druck von 12 bar und einer Temperatur von 80 °C wird hydriert. Die Hydrierzeit beträgt $1\frac{1}{2}$ Stunden. Man erhält in quantitativer Ausbeute 1-Chlor-3-aminobenzol mit einer Reinheit von 99,4 % (gaschromatographische Analyse).

Beispiel 5:

48,0 g 1,2-Dichlor-4-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 12 bar und einer Temperatur von 80 °C wird hydriert. Die Hydrierzeit beträgt 1 Stunde. Man erhält in quantitativer Ausbeute 1,2-Dichlor-4-aminobenzol mit einer Reinheit von 99,7 % (gaschromatographische Analyse).

Beispiel 6:

48,0 g 1,4-Dichlor-2-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 12 bar und einer Temperatur von 80 °C wird hydriert. Die Hydrierzeit beträgt 1 Stunde. Man erhält in quantitativer Ausbeute 1,4-Dichlor-2-aminobenzol mit einer Reinheit von 99,6 % (gaschromatographische Analyse).

Beispiel 7:

48,0 g 1,2-Dichlor-3-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 12 bar und einer Temperatur von 80 °C wird hydriert. Die Hydrierzeit beträgt 1 Stunde. Man erhält in quantitativer Ausbeute 1,2-Dichlor-3-aminobenzol mit einer Reinheit von 99,7 % (gaschromatographische Analyse).

Beispiel 8:

50,5 g 1-Brom-2-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 65 °C wird hydriert. Die Hydrierzeit beträgt $2\frac{1}{2}$ Stunden. Man erhält in quantitativer Ausbeute 1-Brom-2-aminobenzol mit einer Reinheit von 97,8 % (gaschromatographische Analyse).

Beispiel 9:

50,5 g 1-Brom-3-nitrobenzol, 2 g Raney-Nickel (60 % ws.), 1,5 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 65 °C wird hydriert. Die Hydrierzeit beträgt 2 Stunden. Man erhält in quantitativer Ausbeute 1-Brom-3-aminobenzol mit einer Reinheit von 98,7 % (gaschromatographische Analyse).

Beispiel 10:

40,8 g 1-Chlor-2,4-dinitrobenzol, 2 g Raney-Nickel (60 % ws.), 2,3 g N,N′-Dibutylformamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 60 °C wird hydriert. Die Hydrierzeit beträgt $1\frac{1}{4}$ Stunden. Man erhält in quantitativer Ausbeute 1-Chlor-2,4-Diaminobenzol mit einer Reinheit von 97 % (flüssigchromatographische Analyse, <Nachweis als 1-Chlor-2,4-diacetamidobenzol>.

Beispiel 11:

39,8 g 1-Chlor-2-nitrobenzol, 2 g Raney-Nickel (60 % ws.), 1,5 g Formamidinacetat, 100 ml Toluol und

20 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 90 °C wird hydriert. Die Hydrierzeit beträgt 4 Stunden. Man erhält in quantitativer Ausbeute 1-Chlor-2-aminobenzol mit einer Reinheit von 99,9 % (gaschromatographische Analyse).

Beispiel 12:

1,5 g Cyanamid, 1,5 g Essigsäure, 2 g Raney-Nickel (60 % ws.), und 120 ml Methanol werden in einem Autoklaven mit Begasungsrührer eingetragen. Unter 4 bar Wasserstoffdruck und einer Temperatur von 25 °C wird das Cyanamid quantitativ zum Formamidinacetat hydriert. Anschliessend werden 40,8 g 1-Chlor-2,4-dinitrobenzol in den Autoklaven dazugegeben und bei einem Druck von 10 bar und einer Temperatur von 60 °C hydriert. Die Hydrierzeit beträgt 1 Stunde. Man erhält in quantitativer Ausbeute 1-Chlor-2,4-diaminobenzol mit einer Reinheit von 97,5 % (flüssigchromatographische Analyse, <Nachweis als 1-Chlor-2,4-diacetamidobenzol>.

Beispiel 13:

3 g Dicyandiamid, 3 g Essigsäure, 2 g Raney-Nickel (60 % ws.), und 120 ml Methanol werden in einem Autoklaven mit Begasungsrührer eingetragen. Unter 4 bar Wasserstoffdruck und einer Temperatur von 25 °C wird das Dicyandiamid zum Formamidinacetat hydriert. Anschliessend werden 40,8 g 1-Chlor-2,4-dinitrobenzol in den Autoklaven dazugegeben und bei einem Druck von 10 bar und einer Temperatur von 60 °C hydriert. Die Hydrierzeit beträgt $1\frac{1}{4}$ Stunden. Man erhält in quantitativer Ausbeute 1-Chlor-2,4-diaminobenzol mit einer Reinheit von 98 % (flüssigchromatographische Analyse, <Nachweis als 1-Chlor-2,4-diacetamidobenzol>.

**Patentansprüche**

1. Verfahren zur Herstellung von halogenierten aromatischen primären Aminen durch katalytische Hydrierung von halogenierten aromatischen Nitroverbindungen in Gegenwart von Raney-Nickel bei einem Druck von 0,1 bis 100 bar und bei einer Temperatur von 30 bis 150 °C in einem inerten Lösungsmittel und in Gegenwart eines Inhibitors gegen eine Dehalogenierung, dadurch gekennzeichnet, dass der Inhibitor ein Formamidinsalz ist.

2. Verfahren gemäss Anspruch 1, worin das Formamidinsalz in einer Menge von 0,1 bis 30 Mol-% verwendet wird, bezogen auf die Nitro-Verbindung.

3. Verfahren gemäss Anspruch 2, worin das Formamidinsalz in einer Menge von 0,5 bis 15 Mol-% verwendet wird.

4. Verfahren gemäss Anspruch 1, worin das Formamidinsalz der Formel I

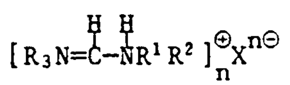

(I)

entspricht, worin $R^1$, $R^2$ und $R^3$ unabhängig voneinander für H stehen, oder lineares oder verzweigtes $C_1$-$C_{12}$-Alkyl, $C_5$- oder $C_6$-Cycloalkyl, $C_6$-$C_{18}$-Alkylcycloalkyl, $C_6$-$C_{10}$-Cycloalkylalkyl, $C_7$-$C_{18}$-Alkylcycloalkylalkyl, wobei das Cycloalkyl 5 oder 6 Ring-C-Atome enthält, $C_6$-$C_{10}$-Aryl, $C_7$-$C_{18}$-Alkaryl, $C_7$-$C_{12}$-Aralkyl oder $C_7$-$C_{18}$-Alkaralkyl oder $R^1$ und $R^2$ zusammen Tetra- oder Pentamethylen oder 3-Oxa-1,5-Pentylen darstellen, X das Anion einer Säure, besonders einer $C_1$-$C_{18}$-Mono- oder -Dicarbonsäure bedeutet und n 1 oder 2 ist.

5. Verfahren gemäss Anspruch 4, worin $R^1$, $R^2$ und $R^3$ unabhängig voneinander H, eine aliphatische oder cycloaliphatische Gruppe sind.

6. Verfahren gemäss Anspruch 4, worin in Formel I $R^1$, $R^2$ und $R^3$ unbhängig voneinander für H oder $C_1$-$C_6$-Alkyl stehen.

**7.** Verfahren gemäss Anspruch 4, worin in Formel I $R^1$, $R^2$ und $R^3$ unabhängig voneinander für H, Methyl oder Ethyl stehen.

**8.** Verfahren gemäss Anspruch 4, worin in Formel I $R^1$, $R^2$ und $R^3$ unabhängig voneinander je für H stehen.

**9.** Verfahren gemäss Anspruch 4, worin n in Formel I für 1 steht.

**10.** Verfahren gemäss Anspruch 4, worin X in Formel I als Anion einer Carbonsäure 1 bis 8 C-Atome enthält.

**11.** Verfahren gemäss Anspruch 4, worin X in Formel I das Anion einer aliphatischen $C_1$-$C_4$-Mono- oder Dicarbonsäure ist.

**12.** Verfahren gemäss Anspruch 11, worin X in Formel I $(COO)_2^{2\ominus}$ , $HCOO^{\ominus}$, $CH_3COO^{\ominus}$, $CH_3CH_2COO^{\ominus}$, $CH_3CH_2CH_2COO^{\ominus}$ oder $CH_2(COO)_2^{2\ominus}$ ist.

**13.** Verfahren gemäss Anspruch 11, worin X für $CH_3COO^{\ominus}$ steht.

**14.** Verfahren gemäss Anspruch 1, worin das Formamidinsalz Formamidinacetat ist.

**15.** Verfahren gemäss Anspruch 1, worin das Raney-Nickel in einer Menge von 0,5 bis 20 Gew.-% verwendet wird, bezogen auf die Nitroverbindung.

**16.** Verfahren gemäss Anspruch 1, worin als Lösungsmittel ein $C_1$-$C_4$-Alkanol alleine oder im Gemisch mit Wasser verwendet wird.

**17.** Verfahren gemäss Anspruch 16, worin das Lösungsmittel Methanol ist.

**18.** Verfahren gemäss Anspruch 1, worin die Temperatur 50 bis 120 °C beträgt.

**19.** Verfahren gemäss Anspruch 1, worin der Druck 1 bis 30 bar beträgt.

**20.** Verfahren gemäss Anspruch 1, worin man das Formamidinsalz durch Hydrierung eines entsprechenden Cyanamids in Gegenwart einer Säure in situ herstellt, und die halogenierte aromatische Nitroverbindung entweder in der gesamten Menge mitvorlegt, oder teilweise mitvorlegt und den Rest nachdosiert.

## Claims

**1.** A process for the preparation of halogenated aromatic primary amines by catalytic hydrogenation of halogenated aromatic nitro compounds in the presence of Raney nickel at a pressure of 0.1 to 100 bar and at a temperature of 30 to 150 °C in an inert solvent and in the presence of an inhibitor against dehalogenation, wherein the inhibitor is a formamidine salt.

**2.** A process according to claim 1, wherein the formamidine salt is used in an amount of 0.1 to 30 mol %, relative to the nitro compound.

**3.** A process according to claim 2, wherein the formamidine salt is used in an amount of 0.5 to 15 mol %.

**4.** A process according to claim 1, wherein the formamidine salt conforms to the formula I

$$[R_3N{=}\overset{H}{C}{-}\overset{H}{N}R^1R^2]_n^{\ominus}X^{n\ominus} \qquad (I)$$

in which $R^1$, $R^2$ and $R^3$, independently of one another, are H, or linear or branched $C_1$-$C_{12}$alkyl, $C_5$cycloalkyl or $C_6$cycloalkyl, $C_6$-$C_{18}$alkylcycloalkyl, $C_6$-$C_{10}$cycloalkylalkyl, $C_7$-$C_{18}$alkylcycloalkylalkyl,

where the cycloalkyl contains 5 or 6 ring carbon atoms, $C_6$-$C_{10}$aryl, $C_7$-$C_{18}$alkaryl, $C_7$-$C_{12}$aralkyl or $C_7$-$C_{18}$alkaralkyl or $R^1$ and $R^2$ together are tetramethylene or pentamethylene or 3-oxa-1,5-pentylene, X is the anion of an acid, in particular of a $C_1$-$C_{18}$ mono- or -dicarboxylic acid, and n is 1 or 2.

**5.** A process according to claim 4, wherein $R^1$, $R^2$ and $R^3$, independently of one another, are H, or an aliphatic or cycloaliphatic group.

**6.** A process according to claim 4, wherein in formula I $R^1$, $R^2$ and $R^3$, independently of one another, are H or $C_1$-$C_6$alkyl.

**7.** A process according to claim 4, wherein in formula I $R^1$, $R^2$ and $R^3$, independently of one another, are H, methyl or ethyl.

**8.** A process according to claim 4, wherein in formula I $R^1$, $R^2$ and $R^3$, independently of one another, are each H.

**9.** A process according to claim 4, wherein n in formula I is 1.

**10.** A process according to claim 4, wherein X in formula I as the anion of a carboxylic acid contains 1 to 8 carbon atoms.

**11.** A process according to claim 4, wherein X in formula I is the anion of an aliphatic $C_1$-$C_4$ mono- or -dicarboxylic acid.

**12.** A process according to claim 11, wherein X in formula I is $(COO)_2^{2\ominus}$ , $HCOO^\ominus$, $CH_3COO^\ominus$, $CH_3CH_2COO^\ominus$, $CH_3CH_2CH_2COO^\ominus$ or $CH_2(COO)_2^{2\ominus}$ .

**13.** A process according to claim 11, wherein X is $CH_3COO^\ominus$.

**14.** A process according to claim 1, wherein the formamidine salt is formamidine acetate.

**15.** A process according to claim 1, wherein the Raney nickel is used in an amount of 0.5 to 20% by weight, relative to the nitro compound.

**16.** A process according to claim 1, wherein the solvent used is a $C_1$-$C_4$alkanol by itself or in a mixture with water.

**17.** A process according to claim 16, wherein the solvent is methanol.

**18.** A process according to claim 1, wherein the temperature is 50 to 120 ° C.

**19.** A process according to claim 1, wherein the pressure is 1 to 30 bar.

**20.** A process according to claim 1, wherein the formamidine salt is prepared by hydrogenation of corresponding cyanamide in the presence of an acid in situ and the halogenated aromatic nitro compound is either initially introduced as well in its total amount or partially introduced as well and the remainder is metered in later.

**Revendications**

**1.** Procédé pour préparer des amines primaires aromatiques halogénées, par hydrogénation catalytique de composés nitro aromatiques halogénés, en présence de nickel de Raney, sous une pression de 0,1 à 100 bar, à une température de 30 à 150 ° C, dans un solvant inerte et en présence d'un inhibiteur protégeant contre une déshalogénation, procédé caractérisé en ce que l'inhibiteur est un sel de formamidine.

**2.** Procédé selon la revendication 1 caractérisé en ce que le sel de formamidine est utilisé en une quantité de 0,1 à 30 % en moles par rapport au composé nitro.

**3.** Procédé selon la revendications 2, caractérisé en ce que le sel de formamidine est utilisé en une quantité de 0,5 à 15 % en moles.

**4.** Procédé selon la revendication 1 caractérisé en ce que le sel de formamidine répond à la Formule I :

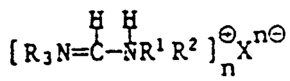

(I)

dans laquelle :

- $R^1$, $R^2$ et $R^3$ représentent chacun, indépendamment l'un de l'autre, l'hydrogène, un alkyle en $C_1$-$C_{12}$ linéaire ou ramifié, un cycloalkyle en $C_5$ ou $C_6$, un alkyl-cycloalkyle en $C_6$-$C_{18}$, un cycloalkyl-alkyle en $C_6$-$C_{10}$, un alkyl-cycloalkyl-alkyle en $C_7$-$C_{18}$ dont la partie cycloalkyle contient, dans son cycle, 5 ou 6 atomes de carbone, un aryle en $C_6$-$C_{10}$, un alkyl-aryle en $C_7$-$C_{18}$, un aralkyle en $C_7$-$C_{12}$ ou un alkyl-aralkyle en $C_7$-$C_{18}$, ou $R^1$ et $R^2$ forment ensemble un radical tétraméthylène, pentaméthylène ou 3-oxa-1,5-pentylène,
- X représente l'anion d'un acide, en particulier d'un acide monocarboxylique ou dicarboxylique en $C_1$-$C_{18}$, et
- n est égal à 1 ou à 2.

**5.** Procédé selon la revendication 4 caractérisé en ce que $R^1$, $R^2$ et $R^3$ représentent chacun, indépendamment l'un de l'autre, l'hydrogène, un radical aliphatique ou un radical cycloaliphatique.

**6.** Procédé selon la revendication 4 caractérisé en ce que les symboles $R^1$, $R^2$ et $R^3$ contenus dans la formule I représentent chacun, indépendamment l'un de l'autre, l'hydrogène ou un alkyle en $C_1$-$C_6$.

**7.** Procédé selon la revendication 4 caractérisé en ce que les symboles $R^1$, $R^2$ et $R^3$ contenus dans la formule I représentent chacun, indépendamment l'un de l'autre, l'hydrogène, un méthyle ou un éthyle.

**8.** Procédé selon la revendication 4 caractérisé en ce que les symboles $R^1$, $R^2$ et $R^3$ contenus dans la formule I représentent chacun, indépendamment l'un de l'autre, l'hydrogène.

**9.** Procédé selon la revendication 4 caractérisé en ce que n, dans la formule I, est égal à 1.

**10.** Procédé selon la revendication 4 caractérisé en ce que le symbole X contenu dans la formule I, en tant qu'anion d'un acide carboxylique, contient de 1 à 8 atomes de carbone.

**11.** Procédé selon la revendication 4 caractérisé en ce que X, dans la formule I, représente l'anion d'un acide monocarboxylique ou dicarboxylique aliphatique en $C_1$-$C_4$.

**12.** Procédé selon la revendication 11 caractérisé en ce que X, dans la formule I, représente $(COO)_2^{2\ominus}$, $HCOO^{\ominus}$, $CH_3COO^{\ominus}$, $CH_3CH_2COO^{\ominus}$, $CH_3CH_2CH_2COO^{\ominus}$ et $CH_2(COO)_2^{2\ominus}$.

**13.** Procédé selon la revendication 11 dans lequel X représente $CH_3COO^{\ominus}$.

**14.** Procédé selon la revendication 1 dans lequel le sel de formamidine est l'acétate de formamidine.

**15.** Procédé selon la revendication 1 dans lequel le nickel de Raney est utilisé en une quantité comprise entre 0,5 et 20 % en poids par rapport au composé nitro.

**16.** Procédé selon la revendication 1 dans lequel on utilise code solvant un alcanol en $C_1$-$C_4$, seul ou en mélange avec de l'eau.

**17.** Procédé selon la revendication 16 dans lequel le solvant est le méthanol.

**18.** Procédé selon la revendication 1 dans lequel la température est comprise entre 50 et 120 ° C.

**19.** Procédé selon la revendication 1 dans lequel la pression est comprise entre 1 et 30 bar.

**20.** Procédé selon la revendication 1 dans lequel le sel de formamidine est préparé in situ, par hydrogénation d'un cyanamide correspondant en présence d'un acide, et le composé nitro aromatique halogéné est soit introduit en totalité dès le départ, soit introduit en partie dès le départ, le reste étant alors ajouté progressivement.